(19) Europäisches Patentamt
European Patent Office
Office européen des brevets

(11) **EP 3 434 278 B1**

(12) **EUROPEAN PATENT SPECIFICATION**

(45) Date of publication and mention of the grant of the patent:
**04.08.2021 Bulletin 2021/31**

(51) Int Cl.:
*A61K 38/48* (2006.01)    *A61P 7/04* (2006.01)
*A61P 7/02* (2006.01)    *A61P 7/06* (2006.01)

(21) Application number: **16894974.1**

(22) Date of filing: **22.04.2016**

(86) International application number:
**PCT/CN2016/079973**

(87) International publication number:
**WO 2017/161621 (28.09.2017 Gazette 2017/39)**

(54) **USE OF ANTIPLATELET THROMBOLYSIN IN THE PREPARATION OF MEDICINE FOR TREATING THROMBOTIC THROMBOCYTOPENIC PURPURA**

VERWENDUNG DES THROMBOZYTENAGGRETATIONSHEMMERS THROMBOLYSIN BEI DER HERSTELLUNG EINES ARZNEIMITTELS ZUR BEHANDLUNG VON THROMBOTISCH-THROMBOZYTOPENISCHER PURPURA

APPLICATIONS DE LA TROMBOLYSINE ANTIPLAQUETTAIRE EN VUE DE LA PRÉPARATION D'UN MÉDICAMENT INDIQUÉ POUR LE TRAITEMENT DE LA MICROANGIOPATHIE THROMBOTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2016 CN 201610180263**

(43) Date of publication of application:
**30.01.2019 Bulletin 2019/05**

(73) Proprietor: **Zhaoke Pharmaceutical (Hefei) Company Limited**
**Hefei, Anhui 230088 (CN)**

(72) Inventors:
• **LI, Xiaoyi**
**Hong Kong Science Park, Shatin (HK)**
• **DAI, Xiangrong**
**Hefei, Anhui 230088 (CN)**

(74) Representative: **de Arpe Fernandez, Manuel**
**Arpe Patentes y Marcas**
**Alcalá, 26, 5ª Planta**
**28014 Madrid (ES)**

(56) References cited:
WO-A1-2014/184352    CN-A- 101 838 323
CN-A- 101 838 323    CN-A- 103 263 662
CN-A- 103 263 663

• **KAMEDA TOMOHIRO ET AL: "Two cases of refractory thrombotic thrombocytopenic purpura associated with collagen vascular disease were significantly improved by rituximab treatment",** CLINICAL RHEUMATOLOGY, ACTA MEDICA BELGICA, BRUXELLES, BE, vol. 26, no. 12, 12 June 2007 (2007-06-12) , pages 2159-2162, XP036400687, ISSN: 0770-3198, DOI: 10.1007/S10067-007-0631-0 [retrieved on 2007-06-12]
• **ALBERTO ALVAREZ-LARRÃ N ET AL: "Newly diagnosed versus relapsed idiopathic thrombotic thrombocytopenic purpura: a comparison of presenting clinical characteristics and response to treatment",** ANNALS OF HEMATOLOGY, SPRINGER, BERLIN, DE, vol. 88, no. 10, 11 February 2009 (2009-02-11), pages 973-978, XP019738403, ISSN: 1432-0584, DOI: 10.1007/S00277-009-0707-9
• **GAO, WEIQIANG et al.: "Progress in thrombotic thrombocytopenic purpura",** Zhong hua nei ke za zhi = Chinese journal of internal medicine, vol. 42, no. 2, 28 February 2003 (2003-02-28), pages 140-142,
• **SADLER, J. E.: "Von Willebrand factor, ADAMTS13, and thrombotic thrombocytopenic purpura",** BLOOD, vol. 112, no. 1, 1 July 2008 (2008-07-01), pages 11-18, XP055422225,

**Sequence Listing**

<110> Zhaoke Pharmaceutical (Hefei) Co., Ltd.

<120> Use of antiplatelet thrombolytic agents in the preparation of a medicament for treatment of thrombotic thrombocytopenic purpura

<160> 2

<170> Patent in version 3.3

<210> 1

<211> 130

<212> PRT <213> Artificial Sequence

<400>1

Asp Val Asp Cys Leu Pro Gly Trp Ser Ala Tyr Asp Gln Ser Cys Tyr
1 5 10 15

Arg Val Phe Lys Leu Leu Lys Thr Trp Asp Asp Ala Glu Lys Phe Cys
20 25 30

Thr Glu Arg Pro Lys Gly Gly His Leu Val Ser Ile Glu Ser Ala Gly
35 40 45

Glu Arg Asp Phe Val Ala Gln Leu Val Ser Glu Asn Lys Gln Thr Asp
50 55 60

Asn Val Trp Leu Gly Leu Lys Ile Gln Ser Lys Gly Gln Gln Cys Ser
65 70 75 80

Thr Glu Trp Thr Asp Gly Ser Ser Val Ser Tyr Glu Asn Phe Ser Glu
85 90 95

Tyr Gln Ser Lys Lys Cys Phe Val Glu Lys Asn Thr Gly Phe Arg Thr
100 105 110

Trp Leu Asn Leu Asn Cys Gly Ser Glu Tyr Ala Phe Val Cys Lys Ser
115 120 125

Pro Pro 130

<210> 2

<211> 125

<212> PRT

<213> Artificial sequence

<400> 2

Gly Phe Cys Cys Pro Leu Arg Trp Ser Ser Tyr Glu Gly His Cys Tyr

1     5          10          15

Leu Val Val Lys Glu Lys Lys Thr Trp Asp Asp Ala Glu Lys Phe Cys

20          25          30

Thr Glu Gln Arg Lys Gly Gly His Leu Val Ser Val His Ser Arg Glu

35          40          45

Glu Ala Asp Phe Leu Val His Leu Ala Tyr Pro Ile Leu Asp Leu Ser

50          55          60

Leu Ile Trp Met Gly Leu Ser Asn Met Trp Asn Asp Cys Lys Arg Glu

65     70     75          80

Trp Ser Asp Gly Thr Lys Leu Asp Phe Lys Ala Trp Ala Lys Thr Ser

85          90          95

Asp Cys Leu Ile Gly Lys Thr Asp Asp Asn Gln Trp Leu Asn Met Asp

100          105          110

Cys Ser Lys Lys His Tyr Phe Val Cys Lys Phe Lys Leu

115          120          125

**Description**

This application claims the priority of Chinese patent application No. 201610180263.4, named "use of antiplatelet thrombolysin in the preparation of a medicament for treatment of thrombotic thrombocytopenic purpura", filed with Chinese Patent Office on March 23, 2016.

**Technical field**

The invention relates to the field of medicine, in particular, to an antiplatelet thrombolysin for use in treating Thrombotic Thrombocytopenic Purpura (TTP).

**Background of the invention**

Thrombotic Thrombocytopenic Purpura (TTP) is a severe diffuse thrombotic microangiopathy, which is characterized by microvascular hemolytic anemia, consumptive reduction of platelet aggregation, and organ damage caused by micro thrombus development (such as kidney, central nervous system, etc.). The pathogenesis thereof is closely related to a vWF protein cleaving enzyme (von Willebrand factor, ADAMTS13). The role of the enzyme is to specifically cleave the von Willebrand factor (vWF) in the blood, so as to ensure the normal hemostatic effect of human body. In the course of TTP, due to a decrease in the activity of the enzyme, vWF is aggregated on vascular endothelial cells and become multiple super large polymers, which in turn can bind to the GPIb receptor on the surface of platelets in the blood stream, mediating adhesion and aggregation of platelet on endothelial cells, finally resulting in thrombosis and further development of TTP.

According to the etiology, TTP can be divided into hereditary TTP and acquired TTP. The difference between the two mechanisms is the different causes of ADAMTS13 enzyme activity decrease. Hereditary patients is due to lack of expression of this enzyme which is in congenital, which is caused by genetic defects. Among acquired TTP patients, the cause of reduced activity is that the patient can produce a kind of auto-antibodies directed against ADAMTS13, which results in enzyme inactivation.

For the above reasons, the most effective treatment for TTP is fresh plasma infusion and plasma exchange, but the drawback thereof is that it relys on objective conditions, and has a very high recurrence rate and mortality of TTP patients. For patients with acquired TTP, immunosuppressive drugs or immunoglobulin etc. can be used to inhibit the production of anti ADAMTS13 auto-antibody, however, this treatment takes effect slowly, the effect may vary from patient to patient, and is not exact. The supplement of ADAMTS13 protein is only effective for congenital TTP patients, because for the acquired TTP patients, the supplemented ADAMTS13 protein will soon be inhibited by auto-antibodies. In patients with TTP, 70%-80% are acquired TTP, therefore, the clinical use of this method is very limited.

**Contents of the invention**

In view of this, the technical problem to be solved by the invention is antiplatelet thrombolysin for use in the treatment of thrombotic thrombocytopenic purpura. It was found in the invention that antiplatelet thrombolysin in blood of TTP patients can restore blood platelet, red blood cell and hemoglobin level, at the same time, reduce the level of lactate dehydrogenase, effectively suppress thrombocytopenia and schistocyte hemolytic anemia, inhibit platelet aggregation, so as to prevent thrombosis and improve the symptoms of TTP.

The antiplatelet thrombolysin (APT) described in the invention is a proteolytic enzyme isolated from Agkistrodon acutus venom, consisting of two polypeptide chains: an alpha chain and a beta chain, wherein the amino acid sequence of the alpha chain is shown as SEQ ID NO.1, and the amino acid sequence of the β chain is shown as SEQ ID NO.2, which is disclosed in patent application 201310228219.2.

Preferably, thrombotic thrombocytopenic purpura is congenital or acquired thrombotic thrombocytopenia purpura.

Preferably, the medicament for treatment of thrombotic thrombocytopenic purpura provided by the disclosure is a chemical or biological preparation.

Preferably, the medicament for treatment of thrombotic thrombocytopenic purpura comprises antiplatelet thrombolysin and a pharmaceutically acceptable excipient.

Preferably, the medicament for treatment of thrombotic thrombocytopenic purpura provided by the disclosure is an oral preparation or injection.

More preferably, the oral preparation for the treatment of thrombotic thrombocytopenic purpura is in the form of tablet, capsule, pill, granule, drop pill, microcapsule or pellet.

More preferably, the mass-volume concentration of antiplatelet thrombolysin in the oral preparation or injection for the treatment of thrombotic thrombocytopenic purpura is 1.8-10$\mu$g/mL.

More preferably, the dose of antiplatelet thrombolysin in the oral preparation or injection for the treatment of thrombotic thrombocytopenic purpura is 0.03-3mg/kg.

The invention provides an antiplatelet thrombolysin medicament for use in the treatment of thrombotic thrombocytopenic purpura.

Antiplatelet thrombolysin can prevent and treat the main symptoms of acquired TTP in baboon model, restore platelet, red blood cell and hemoglobin levels, at the same time, reduce the level of lactate dehydrogenase, effectively suppress the thrombocytopenia and schistocyte hemolytic anemia, so as to achieve the purpose of treatment of TTP. Therefore, the use of the antiplatelet thrombolysin provided by the invention is different from the use disclosed in the prior art, and the mechanism thereof is advantageous than that of the existing means for treating TTP, and can remedy the deficiency of the prior art.

The experiment results showed that, after treatment with antiplatelet thrombolysin, the platelet aggregation inhibition rate in the sample of Platelet Rich Plasma (PRP) in TTP patients was significantly improved compared with that of the control group. These results suggest that antiplatelet thrombolysin has a significant inhibitory effect on platelet aggregation in TTP patients, an effect on prevention of thrombosis and thrombocytopenia, and it has a good prospect of clinical application..

## Description of figures

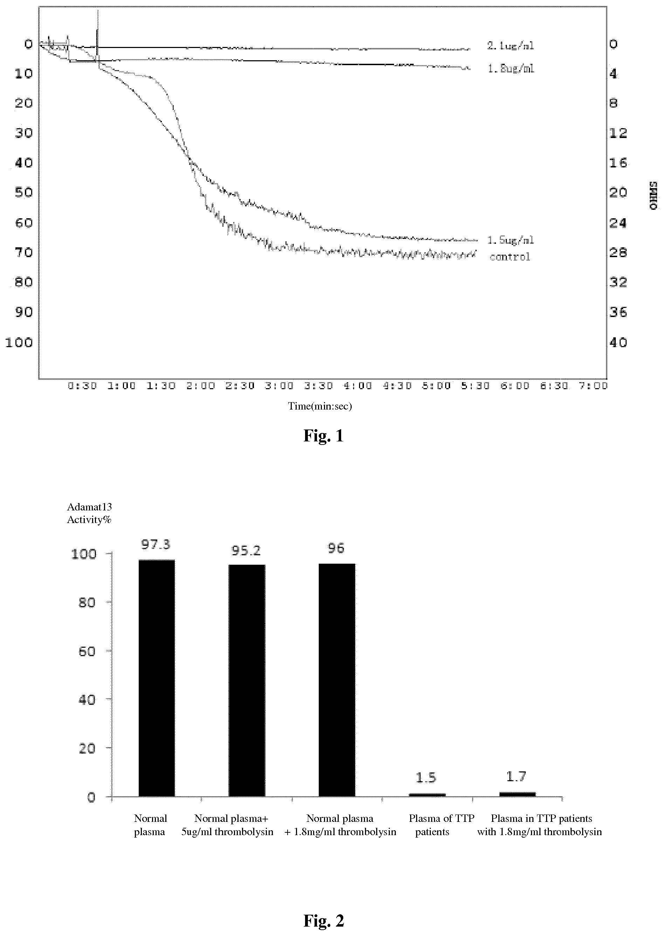

Figure 1 shows the inhibition effect on the antiplatelet thrombolysin in different concentrations on normal human plasma Ristocetin induced platelet aggregation. The green curve represents the platelet aggregation and time curve in blank control group; the purple curve represents the platelet aggregation and time curve after adding 1.5ug/mL of antiplatelet thrombolysin; the blue curve represents platelet aggregation and time curve after adding 1.8ug/mL of antiplatelet thrombolysin; and the black curve represents the platelet aggregation and time curve after adding 2.1ug/mL of antiplatelet thrombolysin.

Figure 2 shows the effect of antiplatelet thrombolysin on the activity of plasma ADAMTS13 in normal humans and TTP patients.

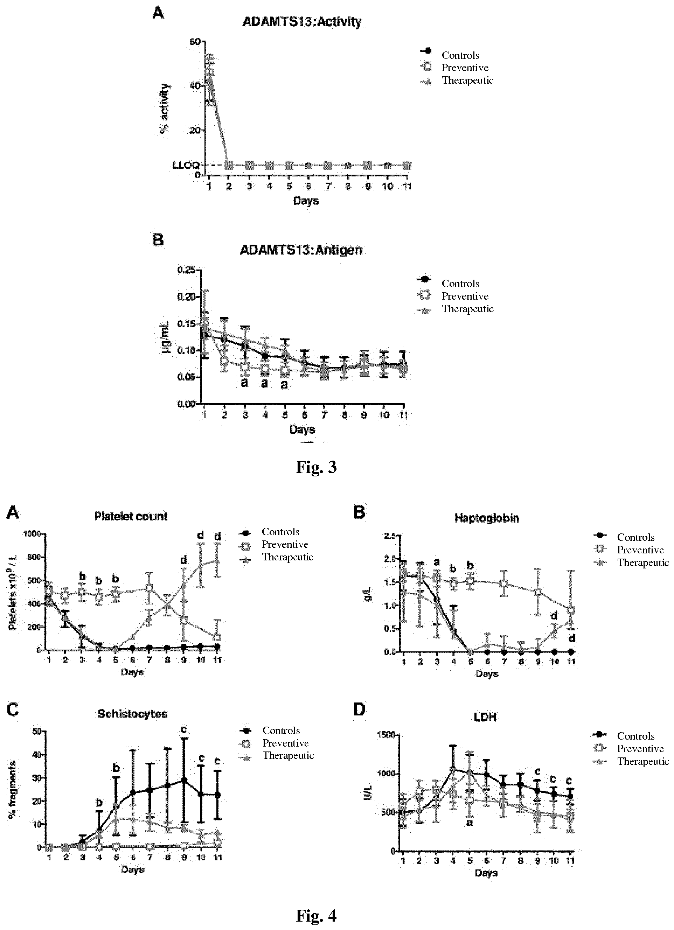

Figure 3 shows the effect of anti ADAMTS13 antibody 3H9 on the concentration and activities of ADAMTS13 antigen in different groups, used for partially reflecting the establishment of TTP model. Figure 3A shows after 3H9 injection, the ADAMTS13 enzymatic activity curves changed over time in control group (the legend is a solid circle), prevention group (the legend is hollow block) and treatment group (legend is solid triangle), Figure 3B shows after 3H9 injection, the ADAMTS13 antigen concentration curves changed over time in the control group (the legend is a solid circle), prevention group (the legend is hollow block) and treatment group (legend is solid triangle).

Figure 4 shows the prevention and treatment effects of antiplatelet thrombolysin on each index of thrombocytopenia and schistocyte hemolytic anemia. Figure 4A shows the curves in platelet count changed over time in the control group (the legend is a solid circle), prevention group (the legend is hollow block) and treatment group (legend is solid triangle); Figure 4B shows the haptoglobin concentration curves changed over time in the control group (the legend is a solid circle), prevention group (the legend is hollow block) and treatment group (legend is solid triangle); Figure 4C shows the schistocyte count curve changed over time in the control group (the legend is a solid circle), prevention group (the legend is hollow block) and treatment group (legend is solid triangle); and Figure 4D shows the lactate dehydrogenase level curves changed over time in the control group (the legend is a solid circle), prevention group (the legend is hollow block) and treatment group (legend is solid triangle).

## Mode of carrying out the invention

The invention provides antiplatelet thrombolysin for use in the treatment of thrombotic thrombocytopenic purpura. The invention can be carried out by those skilled in the art through reading the disclosure herein with appropriate modification of process and parameters. In particular, all similar replacements and modifications are obvious for those skilled in the art, which are deemed to be within the scope of the invention. The method and application of the invention has been described through preferred examples.

The reagents of the invention are all commercial products, and can all be purchased from the market. The following contents will further illustrate the invention in combination with examples:

**Example 1 The inhibitory effect of antiplatelet thrombolysin on platelet aggregation induced by Ristocetin in normal humans and TTP patients *in vitro*.**

1. Sample collection

3 cases of normal blood samples were collected from healthy blood donors. 3 TTP patients were selected from emergency TTP patients. The three patients all had typical clinical "TTP triad", which ADAMTS13 activity in Laboratory testing was lower than 10%. The above samples were all from peripheral venous blood with trisodium citrate anticoagulant.

2. Experimental method

(1) Inhibition test of platelet aggregation (Optical heterometry method):

Whole blood containing 3.2% sodium citrate anticoagulant was taken and centrifuged at 800 PRM for 10min to prepare PRP. The rest of the blood was centrifuged at 3000 PRM for 10min to prepare platelet poor plasma (PPP). The PRP platelet concentration was adjusted to 300 x 109/L with PPP. The change of transmittance in 5 minutes was observed by adding ADP to PRP to a final concentration of 10 umol/L, after the PPP was adjusted to zero. The maximum transmittance was regarded as the maximum aggregation rate of PRP (PAGm).

$$\text{Inhibition rate of platelet aggregation} = \frac{\text{PAGm without Thrombolysin} - \text{PAGm with Thrombolysin}}{\text{PAGm without Thrombolysin}} \times 100\%$$

(2) The test of plasma ADAMTS13 activity (Chemical fluorescence FRET method)

The plasma to be tested was mixed evenly with fluorescence buffer (5mmol/L Bis-Tris, 25mmol/L CaCl2, 0.005% Tween 20, pH 6) at a concentration of 1:25, meanwhile, normal human plasma was used as a standard sample, which was diluted with the above buffer in a ration of 1:12.5, 1:25, 1:50, 1:100, 1:200 and 1:400, as a standard curve. The mixed plasma of normal people in different concentrations and the samples to be tested were put into a 96-well half-skirt plate with 50ul per well, then a certain concentration (2umol/L) fluorescence labeled substrate FRETS-VWF73 with 50ul per well was added into above 96 well plate for enzyme reaction with plasma to be tested. After 5 minutes, the reaction was detected in the microplate. reader The reaction temperature was set at 4 °C, the fluorescence excitation/emission wavelength was 485/530nm, and the frequency was set at one read every 2 minutes, the readings were terminated after 60 minutes. The whole process did not exceed 2 hours. The data of each well at each time point were curve-fitted to obtain the sample reaction curve equation. The slope of the test sample was put into the above log linear regression equation, with ADAMTS13 activity of 1:25 concentration normal mixed plasma as 100%, by comparing with which ADAMTS13 activity of the sample to be measured can be obtained.

3. Experimental result

(1) Inhibition test of platelet aggregation:

When the final concentration of the antiplatelet thrombolysin reached 1.8ug/mL, the inhibition rate on the normal human plasma Ristocetin induced platelet aggregationcould be up to 93%. From the features of the aggregation curve and dose-effect relationship, antiplatelet thrombolysin mainly inhibited "the first phase" of platelet aggregation, that is, inhibits platelet activation and further aggregation by blocking the "outside-in" signaling pathway. The effect of inhibiting aggregation was very effective (Fig. 1).

Platelet aggregation induced by plasma Ristocetin in TTP patients was inhibited by antiplatelet thrombolysin. It was found from the results of the three TTP samples, 1.8ug/mL concentration of antiplatelet thrombolysin was equally effective on inhibiting platelet aggregation in TTP patients (see table below). The following table shows the inhibitory effect of antiplatelet thrombolysin (1.8ug/mL) on platelet aggregation in different populations.

| Sample | Normal | | | TTP | | |
|---|---|---|---|---|---|---|
| | 1# | 2# | 3# | 1# | 2# | 3# |
| Maximum inhibition rate | 93% | 97% | 96% | 91% | 95% | 97% |
| Average | 95.3% | | | 94.3% | | |

(2) The test of plasma ADAMTS13 activity

Antiplatelet thrombolysin of two different concentrations (5 ug/mL, 1.8ug/mL) had no significant effect on plasma ADAMTS13 activity of normal humans or TTP patients (Figure 2).

4. Conclusion

(1) The antiplatelet thrombolysin had obvious inhibition on plasma Ristocetin induced plate aggregation in normal humans or TTP patients. The batch (20130603) product with 1.8ug/mL (final concentration) could achieve the maximum inhibition rate of more than 90%, and there was no difference in inhibition on platelet aggregation in TTP patients and normal humans.

(2) The normal therapeutic concentration of antiplatelet thrombolysin had no significant effect on the ADAMTS 13 activity in normal human plasma, showing that it did not inhibit the behavior of the normal ADAMTS13 cleaving vWF.

**Example 2 Effectiveness experiment for antiplatelet thrombolysin on acquired TTP in baboon model**

1. Experimental design and method

The experiment was divided into three groups, with 4 animals in each group. The first group was used as positive control. From day 0 of the experiment, the animals were injected with anti ADAMTS13 antibody 3H9 at 600ug/kg every 48 hours through femoral vein to establish the TTP model, until day 9 of the experiment, i.e., the end of the experiment. The second group was the preventive group, and the same dose of 3H9 was injected on day 1 and day 3, the 0.3mg/kg antiplatelet thrombolysin was subcutaneously injected on day 1 and day 5 to observe its preventive effect on TTP. The third group was the treatment group, starting from day 0 of the trial, which was injected with anti DAMTS13 antibody 3H9 at 600ug/kg every 48 hours through femoral vein, until day 9, i.e., the end of the experiment, to establish TTP model. Among them, from day 5 to day 11, 0.3mg/kg of antiplatelet thrombolysin was injected subcutaneously every day to observe its therapeutic effect on TTP.
Blood samples were taken every day and hematologic parameters were measured, which include: whole blood cell count, lactate dehydrogenase level, haptoglobin level, schistocyte count and the like. The ADAMTS13 antigen concentration and activity parameters were determined by specific ELISA test.

2. Experimental results

(1) Establishment of TTP model:

The injection of anti ADAMTS13 antibody 3H9 every 48 hours resulted in significant decrease of ADAMTS13 enzyme activity, which also causes the decrease of the concentration of ADAMTS13 antigen (Fig. 3). It directly resulted in thrombocytopenia and hemolytic anemia in animals, platelet count and haptoglobin count were reduced, schistocyte count and lactic dehydrogenase levels were increased (Fig. 4). These are all typical symptoms of TTP, and are also indicators of the success of the model establishment.

(2) Effectiveness of thrombolysin in prevention group:

Compared with the control group, in the prevention group, antiplatelet thrombolysin prevented the reduction of platelet count, and inhibited the occurrence of symptoms including schistocyte hemolytic anemia and abnormal levels of lactate dehydrogenase. No reduction of red blood cell count and hemoglobin concentration were observed in this group of animals (Fig. 4).

(3) Effetiveness of thrombolysin in the treatment group:

In the treatment group, TTP symptoms were observed on t day 5 of the experiment, all indexes were consistent with those of the control group, indicating that the model was well established. Platelet count increased significantly after antiplatelet thrombolysin injection on day 5, and recovered the initial level onday 9, and increased to 180% of the initial level on day 11. schistocyte hemolytic anemia was effectively treated after day 5, the schistocyte count also decreased significantly, but the haptoglobin concentration tended to normalize from day 10 (Fig. 4).

3. Conclusion

Experiments have demonstrated that antiplatelet thrombolysin can prevent and treat the major symptoms of acquired TTP in baboon model, restore platelet, red blood cell and hemoglobin levels, at the same time, reduce the level of lactate dehydrogenase, effectively inhibit thrombocytopenia and schistocyte hemolytic anemia so as to achieve the purpose of the treatment of TTP.

The use of antiplatelet thrombolysin in the preparation of a medicament for the treatment of thrombotic thrombocytopenic purpura provided by the invention has been described through examples. Those skilled in the relevant field can make change or appropriate modification and combination to the use of antiplatelet thrombolysin in the preparation of a medicament for the treatment of thrombotic thrombocytopenic purpura described herein without departing from the contents, spirit and scope of the invention, so as to achieve the technology of the invention.. In particular, all similar replacements and modifications are obvious for those skilled in the art, which are deemed to be included in the spirit, scope and contents of the invention.

## Claims

1. Antiplatelet thrombolysin for use in the treatment of thrombotic thrombocytopenic purpura, wherein the antiplatelet thrombolysin consists of two polypeptide chains: alpha chain and beta chain, the amino acid sequence of the alpha chain is shown as in SEQ ID NO.1, and the amino acid sequence of the beta chain is as shown in SEQ ID NO.2.

2. The antiplatelet thrombolysin for the use of Claim 1, **characterized in that** the thrombotic thrombocytopenic purpura is congenital or acquired thrombotic thrombocytopenic purpura.

3. The antiplatelet thrombolysin for the use of Claim 1, **characterized in that** the medicament is a chemical or biological preparation.

4. The antiplatelet thrombolysin for the use of Claim 1, **characterized in that** the medicament comprises antiplatelet thrombolysin and a pharmaceutically acceptable excipient.

5. The antiplatelet thrombolysin for the use of Claim 1, **characterized in that** the medicament is an oral preparation or an injection.

6. The antiplatelet thrombolysin for the use of Claim 5, **characterized in that** the oral preparation is tablet, capsule, pill, granule, drop pill, microcapsule or micropellet.

7. The antiplatelet thrombolysin for the use of Claim 5, **characterized in that** the mass-volume concentration of the antiplatelet thrombolysin in the oral preparation or injection is 1.8-10 $\mu$g/mL.

8. The antiplatelet thrombolysin for the use of Claim 5, **characterized in that** the dose of the antiplatelet thrombolysin in the oral preparation or injection is 0.03-3mg/kg.

**Patentansprüche**

1. Antithrombozyten-Thrombolysin für die Anwendung in der Behandlung von thrombotische-thrombozytopenische Purpura, wobei das Antithrombozyten-Thrombolysin aus zwei Polypeptidenketten besteht: Alphakette und Betakette, die Aminosäurensequenz der Alphakette wie in SEQ ID NO.1 und die Aminosäurensequenz der Betakette wie in SEQ ID NO.2 gezeigt ist.

2. Das Antithrombozyten-Thrombolysin für die Anwendung gemäss Anspruch 1, **dadurch gekennzeichnet, dass** die thrombotische-thrombozytopenische Purpura eine kongenitale oder erworbene thrombotische-thrombozytopenische Purpura ist.

3. Das Antithrombozyten-Thrombolysin für die Anwendung gemäss Anspruch 1, daduch gekennzeichnet, dass das Medikament ein chemisches oder biologisches Präparat ist.

4. Das Antithrombozyten-Thrombolysin für die Anwendung gemäss Anspruch 1, **dadurch gekennzeichnet, dass** das Medikament ein Antithrombozyten-Thrombolysin und einen pharmazeutisch akzeptierbaren Träger umfasst.

5. Das Antithrombozyten-Thrombolysin für die Anwendung gemäss Anspruch 1, **dadurch gekennzeichnet, dass** das Medikament ein orales Präparat ist oder in Form einer Injektion.

6. Das Antithrombozyten-Thrombolysin für die Anwendung gemäss Anspruch 5, **dadurch gekennzeichnet, dass** das orale Präparat eine Tablette, eine Kapsel, eine Pille, ein Granulat, eine Perle, eine Mikrokapsel oder eine Mikrotablette ist.

7. Das Antithrombozyten-Thrombolysin für die Anwendung gemäss Anspruch 5, **dadurch gekennzeichnet, dass** die volumetrische Massenkonzentration des Antithrombozyten-Thrombolysins in dem oralen Präparat oder der Injektion 1,8-10 µg/ml beträgt.

8. Das Antithrombozyten-Thrombolysin für die Anwendung gemäss Anspruch 5, **dadurch gekennzeichnet, dass** die Dosis des Antithrombozyten-Thrombolysins im oralen Präparat oder der Injektion 0,03-3 mg/kg beträgt.

**Revendications**

1. Thrombolysine antiplaquettaire pour être utilisée dans le traitement du purpura thrombocytopénique thrombotique, où la thrombolysine antiplaquettaire comprend deux chaînes polypeptidiques: alpha et béta; la séquence d'acides aminés de la chaîne alpha est représentée par la SEQ ID NO. 1; et la séquence d'acides aminés de la chaîne béta est représentée par la SEQ ID NO. 2.

2. La thrombolysine antiplaquettaire pour être utilisée conformément à la revendication 1, **caractérisée en ce que** le purpura thrombocytopénique thrombotique est congénital ou acquis.

3. La thrombolysine antiplaquettaire pour être utilisée conformément à la revendication 1, **caractérisée en ce que** le médicament est une préparation chimique ou biologique.

4. La thrombolysine antiplaquettaire pour être utilisée conformément à la revendication 1, **caractérisée en ce que** le médicament contient de la thrombolysine antiplaquettaire et un excipient acceptable sur le plan pharmaceutique.

5. La thrombolysine antiplaquettaire pour être utilisée conformément à la revendication 1, **caractérisée en ce que** le médicament est une préparation à administration par voie orale ou par injection.

6. La thrombolysine antiplaquettaire pour être utilisée conformément à la revendication 5, **caractérisée en ce que** la préparation par voie orale est administrée sous forme de comprimés, capsules, pilules, granulés, gouttes, microcapsules ou micropellets.

**7.** La thrombolysine antiplaquettaire pour être utilisée conformément à la revendication 5, **caractérisée en ce que** la concentration masse/volume de la thrombolysine antiplaquettaire dans la préparation à administration par voie orale ou par injection est de 1,8-10 $\mu$g/mL.

**8.** La thrombolysine antiplaquettaire pour être utilisée conformément à la revendication 5, **caractérisée en ce que** la dose de thrombolysine antiplaquettaire dans la préparation à administration par voie orale ou par injection est de 0,03-3mg/kg.

Fig. 1

Fig. 2

**Fig. 3**

**Fig. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201610180263

- WO 201310228219 A